# EUROPEAN PATENT SPECIFICATION

(11) **EP 1 084 593 B1**
(45) Date of publication and mention of the grant of the patent: **15.08.2007**
(21) Application number: 97941735.9
(22) Date of filing: 29.09.1997
(51) Int. Cl.: H04R 25/00, A61F 11/00, G08B 21/00

(54) **PUBLIC ALARM APPARATUS AND METHOD FOR COCHLEAR IMPLANTS**
VORRICHTUNG UND VERFAHREN ZUR ALARMDURCHSAGE FÜR COCHLEARE IMPLANTATE
APPAREIL ET PROCEDE D'ALARME PUBLIQUE POUR IMPLANTS COCHLEAIRES

(43) Date of publication of application: 21.03.2001
(73) Proprietor: Cochlear Limited, Lane Cove, NSW 2066 (AU)
(72) Inventor: SINGLE, Peter, Lane Cove, NSW 2066 (AU)
(74) Representative: Molyneaux, Martyn William
(86) International application number: PCT/AU1997/000649
(87) International publication number: WO 1999/017585

(56) References cited:
- EP-A1- 0 581 416
- AU-A1- 6 865 596
- US-A- 4 532 930
- US-A- 5 314 453
- US-A- 5 486 814
- US-A- 5 584 869
- DERWENT ABSTRACT, Accession No. 95-016402/03, Class Q17; & GB 2279170 A (NEWTON T) 21 December 1994.

## Description

### BACKGROUND OF THE INVENTION

### A. Field of the Invention

This invention pertains to an apparatus for providing an alarm indicative of a condition of a wearable therapeutic device, such as, for example, a cochlear implant system, as well as to a method related to said apparatus.

### B. Description of the Prior Art

Various devices, such as cochlear implants, are being used to assist persons having a chronic aural disability or impairment which cannot be alleviated by external hearing aids. These devices typically include two components: an external component and an internal component which is implanted into the patient. The external component typically includes a microphone for converting ambient sounds into electrical signals, signal processing means for converting the electrical signals into processed signals, and a signal transmitter for transmitting the processed signals to the internal component. In turn, the internal component includes receiver means for receiving signals from the transmitter, signal processor means for processing the received signals, and stimulating means for stimulating the inner ear of the person, such as the cochlea, in accordance with the received signals. The external component further includes various manual controls for the operation of the device, such as a volume control, battery checking, and so on.

It has been found that for best results, cochlear implant devices should be provided to a patient at the earliest possible age. A problem however occurs if the patient is a small, immature infant. More specifically, with most patients, if the device stops functioning, for example due to a low battery, or a malfunction, the patient can indicate this to a nurse, spouse or other attendant. However, an infant who is essentially deaf and is unable to communicate cannot indicate to anyone that there is a problem, especially since he may be too young to even recognise that there was a problem in the first place.

US 5,584,869 discloses a cochlear implant system having an external component and an internal component controlled by RF signals corresponding to stimulation signals transmitted from the external component to the internal component. The internal component is arranged to generate a feedback signal which is transmitted therefrom to the external component and hence to an external monitor. The feedback signal is distinct from the stimulation signals.

### OBJECTIVES AND SUMMARY OF THE INVENTION

In view of the above-mentioned disadvantages, it is an objective of the present invention to provide a system or apparatus which automatically monitors a cochlear device, and provides either an audible or visual indication to an attendant in case of a problem.

A further objective is to provide a system which can be used easily by a person, without any special instructions or extensive learning period.

Yet a further objective is to provide a device which can be made easily and inexpensively, preferably without the need for expensive additional parts or extensive changes in existing product lines.

Preferably the indicating method and apparatus can be readily used with existing cochlear devices without any modifications.

Other objectives and advantages of the invention shall become apparent from the following description. Briefly, a system for aiding a hearing impaired person constructed in accordance with this invention includes a first external component, located externally to the person, having a microphone for receiving ambient sounds and converting the same into electrical signals, signal processing means for converting the signals and a transmitter for sending the converted signals to an internal component, located internal to the person, through an inductive coupling. This coupling is normally associated with, or produces as a by-product, a radio frequency (RF) signal. Importantly, the present invention further contemplates a second external component, located extend to the patient, being a monitor for monitoring the operation of the first external component. The second external component is adapted to receive and sense an RF signal associated with the inductive coupling between the first external component and the internal component, and to generate an audible or visual indication via an appropriate annunciator as to whether said signal is sensed or not. This second external component is normally worn on a clothing article or pocket by an attendant including a parent, a nurse, or other attendant, so that the latter can determine easily whether the first external component, and preferably the internal component, is operating properly.

Preferably the operation indicative signal is simply leaked RF associated with the transcutaneous RF coupling between the first external component and the internal component. Alternatively the invention envisages that the operation indicative signal may be provided by a second source of RF incorporated into the first external component.

### BRIEF DESCRIPTION OF THE FIGURES

Figure 1 shows a standard prior art cochlear implant device;
Figure 2 shows a monitoring component used to monitor a cochlear implant device such as the one shown in Figure 1; and
Figure 3 shows an alternate embodiment of the invention.
Figure 4 shows a further embodiment of the invention.

### DETAILED DESCRIPTION OF THE INVENTION

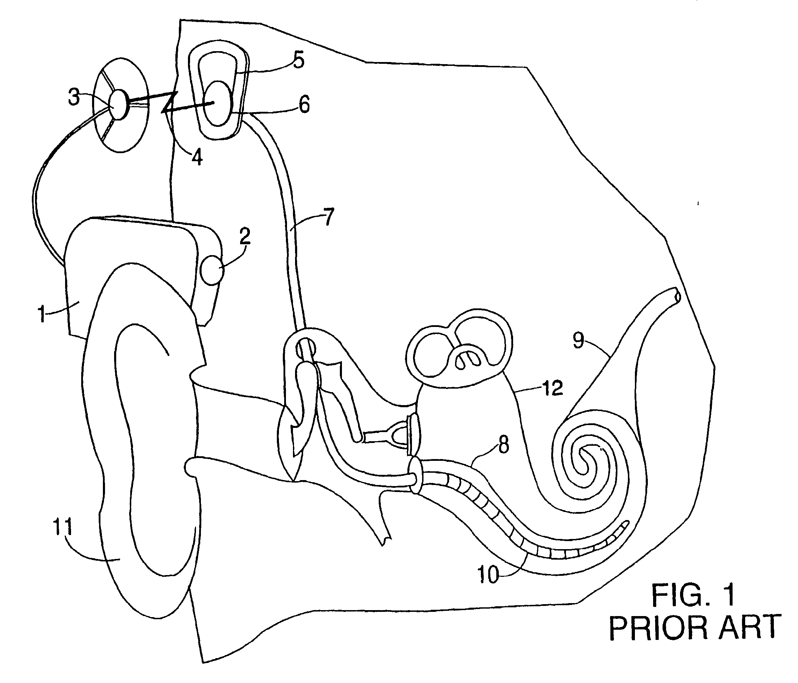

Referring now to the drawings, and more particularly to Figure 1, a typical cochlear implant device consists of an external component which is essentially a speech processor 1 and an internal component including an implanted receiver and stimulator unit 6. The external component 1 includes a microphone 2.

The speech processor 1 is constructed and arranged so that it can fit behind the outer ear 11. Alternatively, it can be worn on the body.

Attached to speech processor 1 is a transmitter coil 3 which transmits-the electrical signals to the internal component via an RF link 4.

The internal or implanted component includes a receive coil 5 for receiving the electrical signals from the coil 3, i.e. RF signals 4. Attached to the receiver coil 5 is the implanted receiver-stimulator 6. A cable 7 extends from the receiver-stimulator 6 to the cochlea 12 and terminates in an electrode array 10. The signals thus received are applied by the array 10 to the nervous tissue of the basilar membrane 8 and thence to the auditory nerve 9. The operation of the device shown in Figure 1 is described for example in U.S. Patent No. 4,532,930.

Under normal conditions, the microphone 2 picks up ambient sounds and converts them into corresponding electrical signals. The speech processor 1 senses these sounds and translates them into corresponding cochlear excitation signals. The excitation signals are then transmitted to the implanted component via channel 4. The receiver-stimulator 6 receives these signals and after suitable translation (if necessary) applies them by means of array 10 to the basilar membrane 8 and thence to auditory nerve 9. Preferably the RF signals are transmitted by an inductive coupling between the coils 3 and 5.

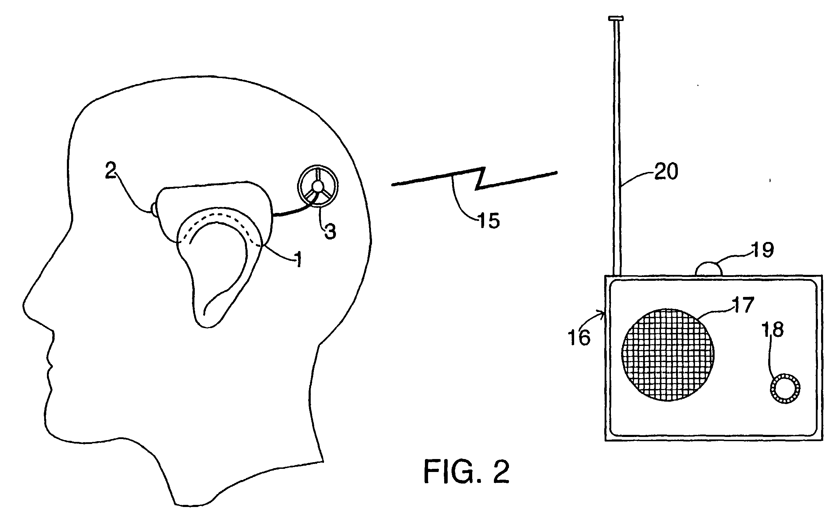

Referring now to Figure 2, an arrangement of an embodiment according to the present invention is depicted, including a monitor 16. The device includes a speaker 17 and may also incorporate an indicator alarm light 19. The monitor is further provided with an on/off-volume controller 18 and an antenna 20.

Importantly, not all the energy of the RF waves 4 (shown in Figure 1) are intercepted by coil 5. Instead some of the energy leaks away, as indicated in Figure 2 at 15. According to a first embodiment the monitor 16 is arranged and constructed so that it receives and senses this leakage and uses it as a confirmation signal that the cochlear device operates satisfactorily.

The simplest method of implementing the invention is to make monitor 16 include a simple radio receiver for detecting the leakage signal 15. The monitor then decides whether the signal 15 is sensed or not. If no signal is sensed, then a sound is emitted by speaker 17 to indicate that either the cochlear implant device is out of range or it has ceased functioning properly. The volume of the sound is controlled by volume controller 18. Alternatively, or in addition, alarm lamp 19 is turned on for the same purpose. The monitor 16 is carried by a parent, guardian, nurse or other attendant associated with the patient.

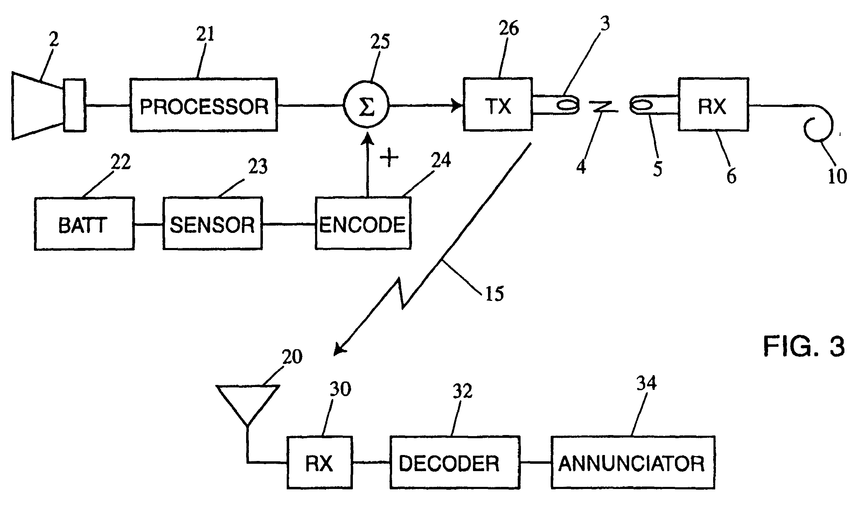

As an additional feature, the speech processor may use an encoder to provide a low battery indication. More particularly, as shown schematically in Figure 3, the speech processor (item 1 of Figure 1) may include a signal processor 21 which receives the electrical signals from microphone 2. The speech processor 1 further includes a battery 22 which provides power to both the speech processor and the internal component.

The condition of the battery 22 is sensed by a sensor 23. The sensor 23 generates an appropriate signal to encoder 24 which generates an indication signal indicative of the battery's condition. This signal is combined with the signal from signal processor 21 by any appropriate means, herein represented by summer 25, for example mixing of the two signals followed by filtering. The resulting signal is transmitted by transmitter 26 to the internal component including coil 5, detected by receiver-stimulator 6 and processed to produce stimulation signals. The stimulation signals are delievered to the patient via cochlear electrode array 10.

Leakage 15 is now detected by receiver 30 through the antenna 20. The received signal is decoded by decoder 32 and a signal dependent on the component of the received signal indicative of the battery condition is fed to an annunciator 34 which may consists of either the speaker 17 or lamp 19. The annunciator 34 is activated if the battery condition indicator signal indicates that the battery 22 is flat.

In this case, the monitor 16 may distinguish between a flat battery condition and the infant being out of range since in the first instance the battery condition signal is present, while in the second instance no signal is sensed at all. Apart from encoding the battery status the signal from encoder 24 may also include a serial number or other data uniquely identifying the speech processor 1. In this manner, several infants may be located in a common area, for example a class room, each infant wearing a special speech processor as described in Figure 3 and the attendant having either a monitor for each infant or a combined monitor adapted to monitor several infants. Of course a disadvantage of this embodiment is that it requires more parts, and in addition the speech processor has to be redesigned to include the battery sensor 23, encoder 24 and summer 25.

According to this embodiment monitor 16 may perform more complicated functions as well, such as determining if the signals from the speech processor 1 are appropriate for the implant, or whether other functions are performed properly by the speech processor.

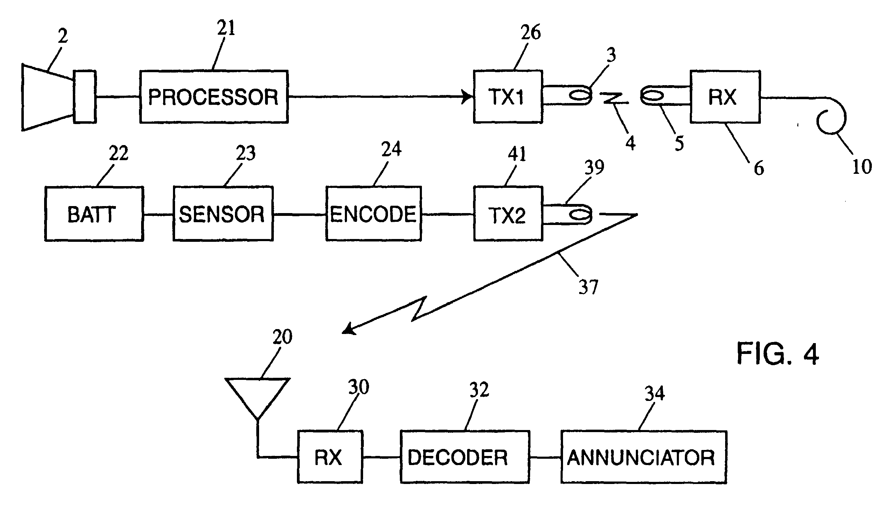

According to another implementation of the invention, depicted in Figure 4, the condition of the battery 22 is sensed as before by a sensor 23. The sensor 23 generates an appropriate signal to encoder 24 which in turn produces a battery condition signal. The battery condition signal is transmitted by second transmitter 41 and transmit antenna 39. The transmitted condition signal 37 is distinct from the transcutaneous coupling RF signal 4. Signal 37 is detected by receiver 30 through antenna 20. The received signal is decoded by decoder 32 and a signal dependent on the component of the received signal indicative of the battery condition is fed to an annunciator 34 which may consist of either the speaker 17 or lamp 19. The operation of the annunciator 34 is exactly the same as that of the annunciator in the embodiment depicted in Figure 3. The operation of the microphone 2, processor 21, first transmitter 26, which generates a transcutaneous coupling signal 4, coils 3 and 5 and the implanted component, including receiver-stimulator 6 and cochlear electrode array 10, is exactly the same as described in the previous embodiment.

It will be readily apparent to those skilled in the art that it is preferable that the condition signal 37 be of a frequency and/or modulation type that does not interfere with transcutaneous coupling signal 4.

Whilst the above described embodiment of Figure 4 is more complex to implement than the earlier embodiment of the invention it has the advantage that the transmitter 31 and antenna 33 can be specifically designed to maximise the operating range between the patient and the monitor. Furthermore in some situations it may be that two or more patients will be situated in close proximity e.g. in a classroom situation. In that case a convenient way of discriminating between alarm signals is to have the transmitter 31 of each each patient's **prosthesis operating on a different frequency. The monitor 16 may be** accordingly modified to scan through the relevant frequencies and upon detecting either a loss of signal or a signal indicative of non-standard operation of a particular cochlear implant device the monitor will issue an alarm identifying the cochlear implant device which is either out of service or operating in a non-standard manner. Alternatively a plurality of monitors, each tuned to monitor a particular patient, may be used, each operating as described previously.

Although the invention has been described with reference to preferred embodiments, it is to be understood that these are merely illustrative of the application of the principles of the invention. Accordingly, the embodiments described in particular should be considered exemplary, not limiting, with respect to the following claims.

## Claims

1. A system for aiding a person having a hearing impairment, said system comprising:
an external component including a microphone (2) for converting ambient sounds into electrical signals;
a signal processor (21) connected to said microphone (2) for processing said electrical signals into stimulation signals;
a transmitter (26; 26, 41) coupled to said signal processor (21) and arranged to transmit RF signals (4, 15) corresponding to said stimulation signals;
an internal component (5, 6, 10) including an internal receiver (6) arranged to receive said RF signals (4) from said transmitter (26; 26, 41), said internal component being arranged to apply pulses to an auditory nerve (9) of said person corresponding to said stimulation signals; and
an external monitor (16) separate from said internal component and having a monitor receiver (30) arranged to receive said RF signals (15) from said transmitter (26; 26, 41), said monitor (16) being arranged to generate an output dependent on said RF signals (15).

2. The system of claim 1 wherein said monitor (16) includes an annunciator (34) arranged to be activated in accordance with said RF signals (15).

3. The system of claim 2 wherein said annunciator (34) is arranged to be activated in the absence of said RF signals (4).

4. The system of claim 2 or claim 3 wherein said annunciator (34) is a speaker (17).

5. The system of claim 2 or claim 3 wherein said annunciator (34) is a lamp (19).

6. The system of any preceding claim wherein said external component is powered by a battery (22) and the transmitter (26, 41) is also arranged to transmit RF signal (37) corresponding to a battery condition and wherein said output is arranged to indicate a condition of said battery.

7. The system of claim 1 wherein said external component includes an encoder (24) for generating an indication signal (37) indicative of a preselected condition and a summer (25) for summing said stimulation signal and said indication signal (37) to generate a combined signal, said transmitter (26) being arranged to transmit RF signals (4, 37) corresponding to said combined signal.

8. The system of claim 7 wherein said monitor (16) includes a decoder (32) for decoding said indication signal (37) from said external component.

9. The system of claim 8 wherein said encoder (24) is arranged to generate a signal including an identifier uniquely identifying said external component and wherein said monitor (16) includes a decoder (32) for recognizing said identifier.

10. The system of any of claims 7 to 9 wherein said indication signal (37) is indicative of a battery condition.

11. The system of any of claims 1 to 6 wherein said monitor receiver (30) is arranged to receive RF leakage signals (15) from a transmitter coil (3) of said transmitter (26), said transmitter coil being inductively coupled to a receiver coil (5) of said internal receiver (6).

12. The system of any of claims 1 to 6 wherein said external component includes an encoder (24) arranged to generate a unique identifier associated with said external component;
said transmitter (26, 41) is arranged to transmit RF signal (4) corresponding to said stimulation signals and said identifier, and
said monitor (16) includes a decoder (32) arranged to decode said identifier.

13. A method of generating a signal indicative of the condition of a therapeutic device, said device having an external component, an internal component (5, 6, 10) and a monitor (16) separate from the internal and external components, wherein said external component transmits to the internal component (5, 6, 10) and said monitor RF signals (4, 15; 4, 37) corresponding to stimulation signals produced by the external component;
said external component transmits a further RF signal (15) corresponding to a battery condition of a battery (22) included in the external component, and
said monitor (16) receives said further RF signal and generates an output dependent on the received RF signals.

14. The method of claim 13 further including the step of activating an annunciator (34) in accordance with said output.

15. The method of claim 13 or claim 14 wherein the external component generates an indication signal indicative of a preselected condition and sums said stimulation signal with said indication signal (37) to generate a combined signal, which is transmitted as said RF signals (4, 15) to said monitor (16).

16. The method of claim 15 wherein said monitor decodes said indication signal from said external component.

17. The method of claim 16 wherein said external component includes an encoder (24) which generates a signal including an identifier uniquely identifying said external component and wherein said monitor (16) decodes said identifier with a decoder (32).

18. The method of any of claims 15 to 17 wherein said indication signal (37) is indicative of said battery condition.

## Patentansprüche

1. System zur Unterstützung einer Person mit einer Gehörschädigung, wobei das System Folgendes aufweist:
- eine externe Komponente, welche ein Mikrofon (2) zur Wandlung von Umgebungsgeräuschen in elektrische Signale umfasst,
- einen Signalprozessor (21), welcher mit dem Mikrofon (2) zur Verarbeitung der elektrischen Signale in Anregungssignale verbunden ist,
- einen Sender (26;26,41), welcher mit dem Signalprozessor (21) gekoppelt ist und derart eingerichtet ist, um RF Signale (4,15), korrespondierend zu den Anregungssignalen zu übertragen,
- eine interne Komponente (5,6,10), beinhaltend einen internen Empfänger (6), ausgelegt zum Empfang der RF Signale (4) von dem Sender (26,41), wobei die interne Komponente derart ausgelegt ist, um Impulse auf einen Gehörnerv (9) von der Person aufzubringen, die mit den Anregungssignalen korrespondieren, und
- einen externen Monitor (16), gesondert von der internen Komponente und mit einem Kontrollempfänger (30), ausgelegt zum Empfang der RF Signale (15) von dem Sender (26;26,41), wobei der Monitor (16) zur Erzeugung eines Ausgangssignals in Abhängigkeit von den RF Signalen (15) ausgelegt ist.

2. System nach Anspruch 1, worin der Monitor (16) ein Meldegerät (34) umfasst, welches derart ausgelegt ist um nach Maßgabe der RF Signale (15) aktiviert zu werden.

3. System nach Anspruch 2, worin das Meldegerät (34) derart ausgelegt ist um bei Abwesenheit der RF Signale (4) aktiviert zu sein.

4. System nach Anspruch 2 oder 3, worin das Meldegerät (34) ein Lautsprecher (17) ist.

5. System nach Anspruch 2 oder 3, worin das Meldegerät (34) eine Lampe (19) ist.

6. System nach einem der vorhergehenden Ansprüche, worin die externe Komponente durch eine Batterie (22) mit Energie versorgt wird und der Sender (26,41) dazu eingerichtet ist RF Signale (37) zu übertragen, korrespondierend mit einem Batteriezustand und worin das Ausgangssignal ausgelegt ist um eine Bedingung der Batterie anzuzeigen.

7. System nach Anspruch 1, worin die externe Komponente einen Kodierer (24) umfasst zur Erzeugung eines Anzeigesignals (37), welches stellvertretend für eine vorausgewählte Bedingung ist und eine Addiereinheit (25) zur Summierung der Anregungssignale und worin das Anzeigesignal (37) zur Generierung eines kombinierten Signals dient, wobei der Sender (26) ausgelegt ist um RF Signale (4,37) zu übertragen, korrespondierend mit dem kombinierten Signal.

8. System nach Anspruch 7, worin der Monitor (16) einen Dekodierer (32) zur Dekodierung des Anzeigesignals (37) von der externen Komponente umfasst.

9. System nach Anspruch 8, worin der Kodierer (24) derart ausgelegt ist um ein Signal zu erzeugen, welches eine eindeutige Kennung zur Kennzeichnung der externen Komponente beinhaltet und worin der Monitor (16) einen Dekcdierer (32) zur Wiedererkennung der Kennung umfasst.

10. System nach einem der Ansprüche 7 bis 9, worin das Anzeigesignal (37) einen Batteriezustand anzeigt.

11. System nach einem der Ansprüche 1 bis 6, worin der Kontrollempfänger (30) derart ausgelegt ist um RF Streusignale (15) von einer Sendespule (3) des Senders (26) zu empfangen, wobei die Sendespule induktiv mit einer Empfangspule (5) des internen Empfängers (6) gekoppelt ist.

12. System nach einem der Ansprüche 1 bis 6, worin die externe Komponente einen Kodierer (24) umfasst, welcher zur Erzeugung einer eindeutigen Kennung ausgelegt ist und welche mit der externen Komponente verknüpft ist,
wobei der Sender (26,41) zur Übertragung von RF Signalen (4) ausgelegt ist, die mit den Anregungssignalen und der Kennung korrespondieren, und
wobei der Monitor (16) einen Dekodierer (32) umfasst, welcher ausgelegt ist zur Dekodierung der Kennung.

13. Verfahren zur Erzeugung eines Signals zur Bezeichnung des Zustands einer therapeutischen Vorrichtung, wobei die Vorrichtung eine externe Komponente umfasst, eine interne Komponente (5,6,10) und einen Monitor (16), gesondert von den internen und externen Komponenten, worin die externe Komponente zu der internen Komponente (5,6,10) und dem Monitor RF Signale (4,15;4,37) überträgt, korrespondierend mit den Anregungssignalen, welche durch die externen Komponenten erzeugt sind,
wobei eine externe Komponente ein weiteres RF Signal (15) überträgt, korrespondierend zu einem Batteriezustand einer Batterie (22), welche in der externen Komponente enthalten ist, und
Wobei der Monitor (16) ein weiteres RF Signal empfängt und ein Ausgangssignal in Abhängigkeit von den empfangenen RF Signalen ausgibt.

14. Verfahren nach Anspruch 13, worin weiterhin der Schritt der Aktivierung eines Meldegeräts (34) entsprechend dem Ausgangssignal umfasst ist.

15. Verfahren nach Anspruch 13 oder 14, worin die externe Komponente ein Anzeigesignal erzeugt, welches bezeichnend ist für eine vorausgewählte Bedingung und weiterhin das Anregungssignal addiert mit dem Anzeigesignal (37), um ein kombinierte Signale zu erzeugen, welche als die RF Signale (4,15) zu dem Monitor (16) übertragen werden.

16. Verfahren nach Anspruch 15, worin der Monitor das Anzeigesignal von der externen Komponente dekodiert.

17. Verfahren nach Anspruch 16, worin die externe Komponente einen Kodierer (24) umfasst, welcher ein Signal erzeugt, dass eine eindeutige Kennung enthält zur Identifizierung der externen Komponente und worin der Monitor (16) die Kennung mittels eines Dekodierers (32) dekodiert.

18. Verfahren nach einem der Ansprüche 15 bis 17, worin das Anzeigesignal (37) den Batteriezustand bezeichnet.

## Revendications

1. Système destiné à assister une personne ayant un trouble de l'audition, ledit système comprenant :
un composant externe comportant un microphone (2) destiné à convertir des sons environnants en signaux électriques ;
un processeur (21) de signaux connecté audit microphone (2) destiné à traiter lesdits signaux électriques pour les transformer en signaux de stimulation ;
un émetteur (26 ; 26, 41) couplé audit processeur (21) de signaux et agencé pour émettre des signaux RF (4, 15) correspondant audits signaux de stimulation ;
un composant interne (5, 6, 10) comportant un récepteur interne (6) agencé pour recevoir lesdits signaux RF (4) à partir dudit émetteur (26 ; 26, 41), ledit composant interne étant agencé pour appliquer des impulsions à un nerf auditif (9) de ladite personne, correspondant audits signaux de stimulation ; et
un moniteur externe (16) séparé dudit composant interne et ayant un récepteur-moniteur (30) agencé pour recevoir lesdits signaux RF (15) provenant dudit émetteur (26 ; 26, 41), ledit moniteur (16) étant agencé pour générer une sortie en fonction desdits signaux RF (15).

2. Système selon la revendication 1, dans lequel ledit moniteur (16) comporte un dispositif avertisseur (34) agencé pour être activé conformément audits signaux RF (15).

3. Système selon la revendication 2, dans lequel ledit dispositif avertisseur (34) est agencé pour être activé en l'absence desdits signaux RF (4).

4. Système selon la revendication 2 ou 3, dans lequel ledit dispositif avertisseur (34) est un haut-parleur (17).

5. Système selon la revendication 2 ou 3, dans lequel ledit dispositif avertisseur (34) est une lampe (19).

6. Système selon l'une quelconque des revendications précédentes, dans lequel ledit composant externe est alimenté par une pile (22) et l'émetteur (26, 41) est également agencé pour émettre un signal RF (37) correspondant à un état de la pile et dans lequel ladite sortie est agencée pour indiquer un état de ladite pile.

7. Système selon la revendication 1, dans lequel ledit composant externe comporte un codeur (24) destiné à générer un signal d'indication (37) indicateur d'un état présélectionné et un sommateur (25) destiné à sommer ledit signal de stimulation et ledit signal d'indication (37) afin de générer un signal combiné, ledit émetteur (26) étant agencé pour transmettre des signaux RF (4, 37) correspondant audit signal combiné.

8. Système selon la revendication 7, dans lequel ledit moniteur (16) comporte un décodeur (32) destiné à décoder ledit signal d'indication (37) provenant dudit composant externe.

9. Système selon la revendication 8, dans lequel ledit codeur (24) est agencé pour générer un signal comportant un identifiant qui identifie de manière unique ledit composant externe et dans lequel ledit moniteur (16) comporte un décodeur (32) destiné à reconnaître ledit identifiant.

10. Système selon l'une quelconque des revendications 7 à 9, dans lequel ledit signal d'indication (37) est indicateur d'un état de la pile.

11. Système selon l'une quelconque des revendications 1 à 6, dans lequel ledit récepteur-moniteur (30) est agencé pour recevoir des signaux de fuite RF (15) provenant d'une bobine émettrice (3) dudit émetteur (26), ladite bobine émettrice étant couplée de manière inductive à une bobine réceptrice (5) dudit récepteur interne (6).

12. Système selon l'une quelconque des revendications 1 à 6, dans lequel ledit composant externe comporte un codeur (24) agencé pour générer un identifiant unique associé audit composant externe ;
ledit émetteur (26, 41) est agencé pour émettre un signal RF (4) correspondant audits signaux de stimulation et audit identifiant ; et
ledit moniteur (16) comporte un décodeur (32) agencé pour décoder ledit identifiant.

13. Procédé pour générer un signal indicateur de l'état d'un dispositif thérapeutique, ledit dispositif ayant un composant externe, un composant interne (5, 6, 10) et un moniteur (16) séparé des composants interne et externe, dans lequel ledit composant externe émet vers le composant interne (5, 16, 10) et ledit moniteur des signaux RF (4, 15 ; 4, 37) correspondant aux signaux de stimulation produits par le composant externe ;
ledit composant externe émet un autre signal RF (15) correspondant à un état de la pile d'une pile (22) incluse dans le composant externe, et
ledit moniteur (16) reçoit ledit autre signal RF et génère une sortie en fonction des signaux RF reçus.

14. Procédé selon la revendication 13, comportant en outre l'étape consistant à activer un dispositif avertisseur (34) conformément à ladite sortie.

15. Procédé selon la revendication 13 ou 14, dans lequel le composant externe génère un signal d'indication indicateur d'un état présélectionné et somme ledit signal de stimulation avec ledit signal d'indication (37) afin de générer un signal combiné, qui est émis en tant que lesdits signaux RF (4, 15) vers ledit moniteur (16).

16. Procédé selon la revendication 15, dans lequel ledit moniteur décode ledit signal d'indication provenant dudit composant externe.

17. Procédé selon la revendication 16, dans lequel ledit composant externe comporte un codeur (24) qui génère un signal comportant un identifiant qui identifie de manière unique ledit composant externe et dans lequel ledit moniteur (16) décode ledit identifiant à l'aide d'un décodeur (32).

18. Procédé selon l'une quelconque des revendications 15 à 17, dans lequel ledit signal d'indication (37) est indicateur dudit état de la pile.
